# EUROPEAN PATENT APPLICATION

(11) **EP 3 530 646 A1**
(43) Date of publication of application: **28.08.2019**
(21) Application number: 18158400.4
(22) Date of filing: 23.02.2018
(51) Int. Cl.: C07C 229/12, C07C 235/12, C11D 1/52, C11D 1/90

(54) **SURFACE ACTIVE COMPOUND**

(71) Applicant: Koninklijke Coöperatie Cosun U.A., 4814 NE Breda (NL)
(72) Inventor: RANOUX, Adeline, 4814 NE Breda (NL); RAAIJMAKERS, Harry, 4814 NE Breda (NL); LAZEROMS, Robert, 4714 DA Sprundel (NL); VAN DER KLIS, Fritz, 6708 WG Wageningen (NL); VAN ES, Daan, 6708 WG Wageningen (NL)
(74) Representative: Nederlandsch Octrooibureau

(57) **Abstract**

The present invention relates to surface active compounds based on polyhydroxy acids. The compounds are of formula (I), where X represents hydrogen or a cation; n is 2, 3 or 4 and Y represents a group of formula (II) or formula (III), wherein at least one of R1, R2 and R3 is a surfactant tail group, and are generally easy to prepare by derivatization of polyhydroxy acids obtained from renewable resources such as, in particular, parenchymal plant materials. The invention relates to the surface active compounds, compositions comprising the same, methods of production and different uses of the surface active compounds.

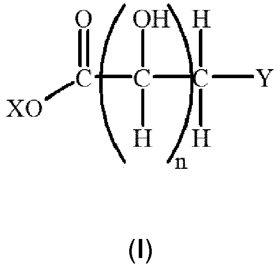

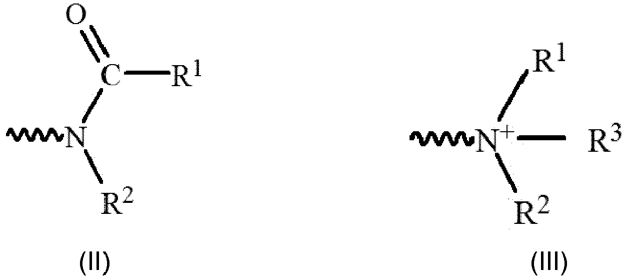

## Description

### Field of the invention

The present invention relates to novel surface active compounds and a process for their production.

### Background of the invention

Surfactants or surface-active agents are used across virtually all industries where they can fulfill a multitude of roles such as, for example, detergent, wetting agent, emulsifier, foaming agent, dispersant, flotation agent etc.

Surfactants are used as "performance chemicals" or "specialty chemicals" in many products and processes and they are produced and consumed in large quantities. The annual surfactant production across different sectors is estimated to be well over 15 billion kilograms. In view of the large-scale production and increasing concerns for the fate of the surfactant after its use, there have been serious and numerous efforts to improve the environmental impact of surfactant production as well as to develop biodegradable surfactants.

A notable effort in this regard is the development of viable synthetic routes towards biodegradable glucose-derived surfactants. WO92/06070 and WO92/06072 describe processes for manufacturing a linear glucamide surfactant comprising reacting an *N*-alkylglucamine, a fatty ester and a catalyst.

Another step towards reducing the environmental impact of surfactant production and use is the development of alkyl polyglycosides which are synthesized from plant- derived glucose and fatty alcohols.

US5633359 describes a process for manufacturing alkyl polyglycosides by reacting a reducing saccharide with excess alcohol followed by thin film evaporation.

In the production of these surfactants components in particular, glycosides are consumed that are (effectively) withdrawn from food supply chains, which may (increasingly) be seen as a disadvantage.

Besides such economic, environmental and/or societal considerations, further technological development in this area is of course also driven by the continuing interest in improvements in surfactant functionality, such as increasing performance, improving compatibility with specific product ranges or even providing entirely new properties or combinations of properties.

The present invention seeks to provide solutions to any or all of the aforementioned problems and objectives.

### Summary of the invention

In view of the aforementioned aims, the present inventors have developed novel surface active compounds, based on polyhydroxy acids.

The compounds of the current invention constitute a novel class of surfactants with distinct and advantageous properties. In accordance with certain embodiments of the invention anionic surfactants are provided. In other embodiments amphoteric surfactants are provided.

In accordance with the invention, surfactants are provided, in particular anionic surfactants, that exhibit superior properties over currently existing (biobased) surface active agents, in terms of e.g. foaming, critical micelle concentration, neutral surface tension, alkaline surface tensions, etc. In embodiments of the invention, biobased surfactants are provided, especially anionic surfactants, that particularly stand out in terms of foaming properties. The surface active compounds of the present invention are generally easy to prepare by derivatization of polyhydroxy acids obtained from renewable resources such as, in particular, parenchymal plant materials. The anionic surface active compounds of some aspects of the present invention are advantageously prepared using fatty esters as a further starting material, avoiding the need to convert the fatty esters to fatty alcohols in order to prepare the surfactant. Processes to produce the compounds of the invention are relatively facile to implement on a commercial scale. Accordingly, the present anionic and amphoteric surface active compounds constitute an attractive (new) class of compounds from the economic and environmental perspective.

In accordance with embodiments of the invention, the surface active compounds provided are biodegradable, which is a further advantage.

The invention thus relates to the surface active compounds, compositions comprising the same, methods of production and different uses of the surface active compounds.

### Detailed description of the invention

A first aspect of the invention concerns compounds of formula (I): 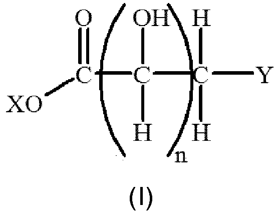 wherein X represents hydrogen or a cation; n is 2, 3 or 4 and Y represents a group of formula (II) or formula (III): 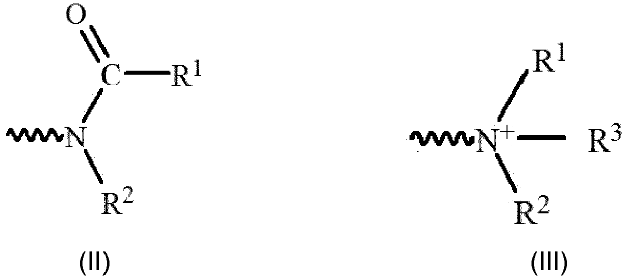 wherein R¹, R² and R³ independently represent one of hydrogen, a C₁-C₄ alkyl, a C₁-C₄ alcohol or a surfactant tail group, with the proviso that at least one of R¹, R² and R³ represents a surfactant tail group.

In preferred embodiments, a compound according to formula (I) is provided wherein Y represents a compound of formula (II) and one of R¹ and R², preferably R¹, represents a surfactant tail group, while the other of R¹ and R², preferably R² represents hydrogen, a C₁-C₄ alkyl or a C₁-C₄ alcohol.

In embodiments of the invention, a compound according to formula (I) is provided wherein Y represents a compound of formula (III) and one of R¹, R² and R³ represents a suitable surfactant tail group, while the others of R¹, R² and R³ represents hydrogen, a C₁-C₄ alkyl or a C₁-C₄ alcohol.

In embodiments a compound according to formula (I) is provided wherein Y represents a group of formula (III) and two of R¹, R² and R³ represent a surfactant tail group, while the other of R¹, R² and R³ represents hydrogen, a C₁-C₄ alkyl or a C1-C4 alcohol.

In embodiments a compound according to formula (I) is provided wherein Y represents a group of formula (III) and all three of R¹, R² and R³ represent a surfactant tail group.

In preferred embodiments of the invention, the surfactant tail group represented by at least one of R¹, R² and R³ is an optionally substituted branched, unbranched, or cyclic group having from 5 to 30 carbon atoms, such as at least 6 carbon atoms, at least 7 carbon atoms, at least 8 carbon atoms, at least 9 carbon atoms, at least 10 carbon atoms, at least 11 carbon atoms, at least 12 carbon atoms, at least 13 carbon atoms, at least 14 carbon atoms or at least 15 carbon atoms and/or up to 28 carbon atoms, up to 26 carbon atoms, up to 25 carbon atoms, up to 24 carbon atoms, up to 23 carbon atoms, up to 22 carbon atoms, up to 21 carbon atoms, or up to 20 carbon atoms.

The surfactant tail group represented by at least one of R¹, R² and R³ may be saturated or may comprise one or more unsaturated moieties, such as alkenyl or alkynyl. That is, the group represented by at least one of R¹, R² and R³ may comprise one or more carbon-to-carbon double bonds and/or one or more carbon-to-carbon triple bonds. Additionally, the surfactant tail group represented by at least one of R¹, R² and R³ may comprise cyclic moieties, i.e. cycloalkyl, either within the main carbon chain or branched therefrom. The surfactant tail group represented by at least one of R¹, R² and R³ may comprise branching moieties, e.g., alkyl, alkenyl, trialkylsilyl, phenyl, and substituted phenyl. The surfactant tail group represented by at least one of R¹, R² and R³ may be a hydrocarbyl (i.e., the entirety of the group comprises only carbon and hydrogen) or the group may comprise heteroatom substituents, such as halide atoms (e.g., fluoride, chloride, and bromide) bonded to carbon atoms. In some embodiments, the surfactant tail group represented by at least one of R¹, R² and R³ contains at least two fluoride atoms, such as at least four fluoride atoms. In some embodiments, the surfactant tail group represented by at least one of R¹, R² and R³ is perfluorinated, meaning that all hydrogen atoms along the carbon chain are replaced with fluoride atoms.

In specific embodiments, the surfactant tail group represented by at least one of R¹, R² and R³ comprises at least one carbon-to-carbon double bond. In certain embodiments, the surfactant tail group represented by at least one of R¹, R² and R³ comprises at least two carbon-to-carbon double bonds, wherein the carbon-to-carbon double bonds are separated by at least one methylene group, such as one methylene group, two methylene groups, three methylene groups, or more.

In a specific embodiment, the surfactant tail group represented by at least one of R¹, R² and R³ is selected from:
- branched or straight chain C₅-C₂₄ alkyl groups;
- branched or straight chain mono-unsaturated C₅-C₂₄ alkenyl groups;
- branched or straight chain poly-unsaturated C₅-C₂₄ alkenyl groups;
- alkylbenzene groups comprising a C₈-C₁₅ alkyl;
- alkenylbenzene groups comprising a C₈-C₁₅ alkenyl;
- alkylnaphthalene groups comprising a C₃-C₁₅ alkyl;
- alkenylnaphthalene groups comprising a C₃-C₁₅ alkenyl,
- alkylphenol groups comprising a C₈-C₁₅ alkyl;
- alkenylphenol groups comprising a C₈-C₁₅ alkenyl; and
- (poly)oxyethylene alkyl ethers comprising an alkyl chain with *n* methylene groups and a (poly)oxyethylene part with m oxyethylene units wherein n is an integer from 5-22, preferably 5-15, more preferably 5-12 and m is an integer from 1-50, preferably 1-25, more preferably 1-15, most preferably 1-10.

In embodiments of the invention, compounds of formula (I) are provided as defined herein wherein the surfactant tail group represented by at least one of R¹, R² and R³ is a branched or straight chain C₅-C₂₄ alkyl group.

In embodiments of the invention, compounds of formula (I) are provided as defined herein wherein the surfactant tail group represented by at least one of R¹, R² and R³ is a branched or straight chain mono-unsaturated C₅-C₂₄ alkenyl group.

In embodiments of the invention, compounds of formula (I) are provided as defined herein wherein the surfactant tail group represented by at least one of R¹, R² and R³ is a branched or straight chain poly-unsaturated C₅-C₂₄ alkenyl group.

In embodiments of the invention, compounds of formula (I) are provided as defined herein wherein the surfactant tail group represented by at least one of R¹, R² and R³ is an alkylbenzene group comprising a C₈-C₁₅ alkyl.

In embodiments of the invention, compounds of formula (I) are provided as defined herein wherein the surfactant tail group represented by at least one of R¹, R² and R³ is a alkenylbenzene group comprising a C₈-C₁₅ alkenyl.

In embodiments of the invention, compounds of formula (I) are provided as defined herein wherein the surfactant tail group represented by at least one of R¹, R² and R³ is an alkylnaphthalene residue comprising a C₃-C₁₅ alkyl.

In embodiments of the invention, compounds of formula (I) are provided as defined herein wherein the surfactant tail group represented by at least one of R¹, R² and R³ is an alkenylnaphthalene residue comprising a C₃-C₁₅ alkenyl.

In embodiments of the invention, compounds of formula (I) are provided as defined herein wherein the surfactant tail group represented by at least one of R¹, R² and R³ is an alkylphenol group comprising a C₈-C₁₅ alkyl.

In embodiments of the invention, compounds of formula (I) are provided as defined herein wherein the surfactant tail group represented by at least one of R¹, R² and R³ is an alkenylphenol groups comprising a C₈-C₁₅ alkenyl.

In embodiments of the invention, compounds of formula (I) are provided as defined herein wherein the surfactant tail group represented by at least one of R¹, R² and R³ is a (poly)oxyethylene alkyl ethers comprising an alkyl chain with *n* methylene groups and a (poly)oxyethylene part with m oxyethylene units wherein n is an integer from 5-22, preferably 5-15, more preferably 5-12 and m is an integer from 1-50, preferably 1-25, more preferably 1-15, most preferably 1-10.

In certain preferred embodiments, compounds of formula (I) are provided as defined herein wherein the surfactant tail group represented by at least one of R¹, R² and R³ is the residue of a C₆-C₂₄ fatty acid, preferably the residue of a fatty acid selected from the group consisting of caproic acid, caprylic acid, capric acid, lauric acid, lauroleic acid, myristic acid, myristoleic acid, palmitic acid, palmitoleic acid, stearic acid, oleic acid, linoleic acid, linolenic acid, ricinoleic acid, arachidic acid, arachidonic acid, gadoleic acid, erucic acid, behenic acid and lignoceric acid.

In certain embodiments of the invention, at least one of R¹, R² and R³ represents a group selected from hydrogen, methyl, ethyl, n-propyl, isopropyl, n-butyl, sec-butyl, isobutyl or *tert*-butyl, preferably from hydrogen, methyl, ethyl, *n-*propyl and isopropyl, more preferably from hydrogen, methyl and ethyl, most preferably from hydrogen and methyl. In formula (I) as defined herein X represents hydrogen or a cation. It will immediately be understood by those skilled in the art, based on the present teachings, that the compounds of the invention can be provided in free acid form, in the form of a salt, typically a base addition salt, in the form of a mixture of different salts or in the form of a mixture of the free acid and one or more salt forms. In embodiments of the invention X represents hydrogen. In embodiments of the invention X represents a cation. In principle, the invention is not limited to any specific (group) of salts and X can represent any cation. In certain embodiments, it may be preferable that the cation is selected from the group consisting of alkaline metal ions, alkaline earth metal ions and quaternary ammonium cations represented by the formula NRR'R"R"' wherein R, R', R" and R"' are independently selected from the group of H, C₁-C₆ alkyls and C₁-C₆ hydroxyalkyls. Alternatively, the quaternary ammonium cation represented by the formula NRR'R"R"' may result from an amine which was employed in the synthesis of the compound in accordance with formula (I). Accordingly, in embodiments X is a quaternary ammonium cation represented by the formula NRR'R"R"' wherein at least one of R, R' and R" and R"' represents a surfactant tail group, typically a surfactant tail group as defined herein elsewhere, while the others of R, R' and R" and R"' independently represent one of hydrogen, a C₁-C₄ alkyl or a C₁-C₄ alcohol. In embodiments, X is a quaternary ammonium cation represented by the formula NRR'R"R"' wherein one or more of R, R' and R" and R"' represent a surfactant tail group identical to at least one of R¹, R² and R³, as defined herein, while the others of R, R' and R" and R"' independently represent one of hydrogen, a C₁-C₄ alkyl or a C₁-C₄ alcohol. In embodiments, X is a quaternary ammonium cation represented by the formula NRR'R"R"' wherein at least one of R, R' and R" and R"' is selected from:
- branched or straight chain C₅-C₂₄ alkyl groups;
- branched or straight chain mono-unsaturated C₅-C₂₄ alkenyl groups;
- branched or straight chain poly-unsaturated C₅-C₂₄ alkenyl groups;
- alkylbenzene groups comprising a C₈-C₁₅ alkyl;
- alkenylbenzene groups comprising a C₈-C₁₅ alkenyl;
- alkylnaphthalene groups comprising a C₃-C₁₅ alkyl;
- alkenylnaphthalene groups comprising a C₃-C₁₅ alkenyl,
- alkylphenol groups comprising a C₈-C₁₅ alkyl;
- alkenylphenol groups comprising a C₈-C₁₅ alkenyl; and
- (poly)oxyethylene alkyl ethers comprising an alkyl chain with *n* methylene groups and a (poly)oxyethylene part with m oxyethylene units wherein n is an integer from 5-22, preferably 5-15, more preferably 5-12 and m is an integer from 1-50, preferably 1-25, more preferably 1-15, most preferably 1-10
while the others of R, R' and R" and R"' independently represent one of hydrogen, a C₁-C₄ alkyl or a C₁-C₄ alcohol.

In formula (I) as defined herein n is 2, 3, or 4, preferably n is 3 or 4, more preferably n is 4.

In preferred embodiments the moiety represented by formula (I'): 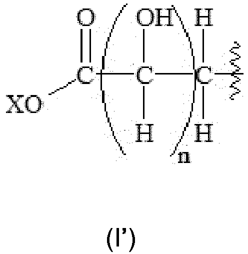 represents a uronic acid residue, preferably the residue of a uronic acid selected from the group consisting of hexuronic acids and penturonic acids; preferably from the group consisting of hexuronic acids; preferably from the group consisting of galacturonic acid, iduronic acid, mannuronic acid and guluronic acid; most preferably galacturonic acid.

In preferred embodiments the stereochemistry of the uronic acid residue which may be present in the compound is the same as the stereochemistry of the D isomer of the corresponding uronic acid.

Hence, in embodiments of the invention compounds of formula (I) are provided wherein: X represents hydrogen or a cation; n is 4; Y represents a compound of formula (II); R¹ represents a C₅-C₂₄ alkyl or alkenyl, preferably the residue of a C₆-C₂₄ fatty acid, preferably the residue of a fatty acid selected from the group consisting of caproic acid, caprylic acid, capric acid, lauric acid, lauroleic acid, myristic acid, myristoleic acid, palmitic acid, palmitoleic acid, stearic acid, oleic acid, linoleic acid, linolenic acid, ricinoleic acid, arachidic acid, arachidonic acid, gadoleic acid, erucic acid, behenic acid and lignoceric acid; and R² represents hydrogen or methyl.

In embodiments of the invention, compounds are provided that are biodegradable. The term "biodegradable" as used herein refers to the fact that the compounds decompose under environmental influences in an appropriate and demonstrable timespan. The degradation of the polymer may be partial or complete and may take place due to hydrolysis and/or oxidation, and also due to the action of microorganisms such as bacteria, yeasts, fungi and algae. In addition, enzymatic degradation is also possible. In some embodiments of the invention, compounds are provided that are readily biodegradable as defined in the OECD Guidelines for the Testing of Chemicals, section 3.

In another aspect of the invention, there is provided a composition or product comprising the compound of the present invention. Compounds of the present invention confer desirable surfactant properties in a large and diverse range of products. The compounds of the present invention may confer beneficial properties already when used in low relative amounts. On the other hand, embodiments are envisaged, wherein the surface active compound of the present invention constitutes a major ingredient. Hence, the relative amount of the compound of formula (I) in a product according to the present invention may vary over a wide range.

In embodiments of the invention, a composition is provided comprising the compound of formula (I) in an amount within the range of 0.05-99 wt.%, based on the total weight of the product, e.g. in an amount of at least 0.1 wt.%, at least 0.2 wt.%, at least 0.3 wt.%, at least 0.4 wt.%, at least 0.5 wt.%, at least 1 wt.%, at least 2 wt.%, at least 5 wt.% or at least 10 wt.% and/or in an amount of up to 50 wt.%, up to 25 wt.%, up to 20 wt.%, up to 15 wt.% or up to 10 wt.%. In embodiments of the invention, a composition is provided comprising the surface active compound in an amount within the range of 0.1-50 wt.%, 0.2-25 wt.%, 0.3-20 wt.%, 0.4-15 wt.% or 0.5-10 wt.%.

In preferred embodiments, the composition or product is selected from the group consisting of pharmaceuticals, neutraceuticals, feed, food, cosmetics, detergents, fabric softeners, soaps, paints, adhesives, inks, anti-fogs, agrochemical products, herbicides, insecticides, biocides, cosmetics, shampoos, hair conditioners, toothpastes, coatings, drilling fluids, oilfield chemicals, emulsion polymerization systems and ferrofluids. More preferably, the product is selected from laundry detergent products, dishwashing products, personal care products, and hard surface cleaning products.

In embodiments of the invention, a composition as defined herein is provided, wherein the composition is a liquid, cream, gel, powder, spray, suspension, slurry, emulsion, lubricant or tablet.

In embodiments of the invention, a composition as defined herein is provided, wherein the composition comprises the compound of formula (I) in conjunction with one, two, three, four or more component(s) selected from the group consisting of solvents, cosolvents, fragrance, softness extenders, other surfactants or emulsifiers, abru-redisposition agents, sequesterants, antiadherents, binders, coatings, colouring agents, disintegrants, flavors, glidants, lubricants, preservatives, sorbents, sweeteners, fillers, flow regulating agents, bulking agents, glycerides, glycols, monoesters of diols, wax, hydrophobic carriers, wetting agents, thickeners, chelating agents, abrasive agents, non-ionic surfactants and ionic surfactants is provided.

In embodiments of the invention, a composition as defined herein is provided, wherein the composition is a laundry detergent product, comprising the surface active compound of the present invention, in conjunction with one or more ingredients selected from the group consisting of phosphates, bleaching agents, optical brighteners, ionic surfactants, non-ionic surfactants, enzymes, alkaline salts, anti-foaming agents, complexing agents, perfumes, anti-caking agents, starches, gelling agents, emulsifiers, dispersing agents, dye transfer inhibitors, fabric softeners, colorants, etc. In preferred embodiments, a composition as defined herein is provided, wherein the composition is a laundry detergent product, comprising the surface active compound of the present invention as a cosurfactant. In embodiments, this may be achieved by incorporating the surface active compound of the present invention in an amount of less than 50 wt.% by weight of all surfactants in the composition, e.g. in an amount of less than 20 wt.%, less than 10 wt.%, less than 5 wt.%, less than 1 wt.%.

In embodiments of the invention, a composition as defined herein is provided, wherein the composition is a dishwashing product, comprising the surface active compound of the present invention, in conjunction with one or more ingredients selected from the group consisting of phosphates, bleaching agents, ionic surfactants, non-ionic surfactants, enzymes, alkaline salts, anti-foaming agents, complexing agents, perfumes, anti-caking agents, starches, gelling agents, emulsifiers, dispersing agents, sand etc.

In embodiments of the invention, a composition as defined herein is provided, wherein the composition is a personal care product, comprising the surface active compound of the present invention, in conjunction with one or more ingredients selected from the group consisting of hydrophobic carriers, cream bases, opacifiers, preservatives, chelating agents, emollients, emulsifiers, neutralizers, humectants, dyes, quenchers, proteins, thickeners, UV filters, vitamins, solubilizers, solvents, perfumes etc. By the term "personal care" is meant products such as skin cleansers, hair treatments (e.g. shampoos, mousses and conditioners), depilatories, skin lightening products, and leave-on skin lotions and creams. These products may be delivered from wipes (e.g. nonwoven substrates), liquids, gels, pumps or stick format.

In embodiments of the invention, a composition as defined herein is provided, wherein the composition is a hard surface cleaning product, comprising the surface active compound of the present invention, in conjunction with one or more ingredients selected from the group consisting of chelating agents, other surfactants, wetting agents, solvents, diluents, acids, disinfectants, hydrophilic polymers, solvents, abrasives, inorganic absorbent materials, bleaching agents, perfume etc.

In embodiments of the invention, a composition as defined herein is provided, wherein the composition is an agrochemical product, comprising the surface active compound of the present invention, in conjunction with one or more ingredients selected from the group consisting of pesticides, insecticides, fungicides, fertilizer compositions.

In embodiments of the invention, a composition as defined herein is provided, wherein the composition is a pharmaceutical/medicinal product, comprising the surface active compound of the present invention, in conjunction with one or more active pharmaceutical ingredients ('APIs'), such as the compounds included in the Anatomical Therapeutic Chemical (ATC) classification system, maintained by the World Health Organization.

In embodiments of the invention, a composition as defined herein is provided, wherein the composition is a nutraceutical or food supplement product, comprising the polyhydroxy acid amide of the present invention, in conjunction with one or more physiologically active ingredients, such as vitamins, minerals, trace elements, and/or one or more food-grade excipients, such as a compound which is recognized by the U.S. Food & Drug administration as GRAS (Generally Recognized as safe). In some embodiments of the invention said physiologically active ingredient is poorly water-soluble.

In embodiments of the invention, a composition as defined herein is provided, wherein the composition is a descaling product, comprising the surface active compound of the present invention, in conjunction with one or more ingredients selected from the group consisting of descaling agents, complexing agents, chelating agents, binders, fillers, acidic compounds such as hydrochloric acid, acetic acid, citric acid, glycolic acid, formic acid, phosphoric acid and sulfamic acid.

In embodiments of the invention, a composition as defined herein is provided, wherein the composition is a chlorine product, comprising the surface active compound of the present invention, in conjunction with one or more ingredients selected from the group of chlorine compounds, such as chlorinated isocyanurates, hypochlorite salts, chlorine dioxide, chloride of lime; etc.

In embodiments of the invention, a composition as defined herein is provided, wherein the composition is a mineral product, comprising the surface active compound of the present invention, in conjunction with one or more ingredients selected from the group consisting of minerals of calcium, phosphorus, potassium, sodium, iron, cobalt, copper, zinc, manganese, molybdenum, iodine, and selenium, bromine, arsenic, nickel, fluorine, boron, lithium, strontium.

In embodiments of the invention, a composition as defined herein is provided, wherein the composition is a bleach product, comprising the surface active compound of the present invention, in conjunction with one or more ingredients selected from the group consisting of chlorine-based bleaches, peroxide-based bleaches, reducing bleaches, peracetic acid, ozone and combinations thereof. Chlorine-based bleaches may comprise hypochlorite compounds such as calcium hypochlorite, chlorine dioxide, etc. Peroxide-based bleaches may comprise hydrogen peroxide, sodium percarbonate and sodium perborate. Reducing bleaches may comprise sodium dithionite and sodium oxymethylene sulfoxylate.

In embodiments of the invention, a composition comprising the compound of formula (I) is provided, wherein the composition comprises an aqueous phase, typically liquid detergent formulations, shampoos, shower gels etc. In embodiments of the invention, a composition as defined herein is provided, having a water content within the range of 5-90 wt.%, based on the total weight of the composition, such as a water content of at least 5 wt.%, at least 10 wt.%, at least 20 wt.%, at least 30 wt.% or at least 40 wt.% and/or a water content of up to 90 wt.%, up to 80 wt.% or up to 70 wt.%.

In embodiments of the invention, a composition comprising the compound of formula (I) is provided, wherein the composition further comprises at least one further component, which further component is poorly water-soluble. In some embodiments of the invention said at least one further component has a water solubility of less than 1 g/ml at a temperature of 20 °C, e.g. a solubility of less than 0.1 g/ml, less than 0.01 g/ml or less than 0.001 g/ml. In embodiments of the invention, a composition as defined herein is provided, wherein the content of said further component is within the range of 0.0001-5 wt.%, based on the total weight of the composition, such as at least 0.0001 wt.%, at least 0.001 wt.%, at least 0.01 wt.%, at least 0.1 wt.% and/or up to 5 wt.%, up to 1 wt.%, up to 0.5 wt.%, up to 0.2 wt.%.

Reference is made to substances, components, or ingredients in existence at the time just before first contacted, formed in situ, blended, or mixed with one or more other substances, components, or ingredients in accordance with the present disclosure. A substance, component or ingredient identified as a reaction product, resulting mixture, or the like may gain an identity, property, or character through a chemical reaction or transformation during the course of contacting, in situ formation, blending, or mixing operation if conducted in accordance with this disclosure with the application of common sense and the ordinary skills of an average chemist. The transformation of chemical reactants or starting materials to chemical products or final materials is a continually evolving process, independent of the speed at which it occurs. Accordingly, as such a transformative process is in progress there may be a mix of starting and final materials, as well as intermediate species. Unless otherwise indicated herein, definitions of (relative) amounts of components concern the composition as is.

In another aspect, the invention concerns a method of producing a compound of formula (I) as defined herein wherein Y represents a group of formula (II), the method comprising:
a) providing a polyhydroxy acid of formula (IV), 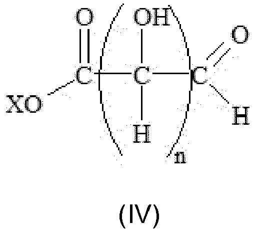 Wherein X and n have the same meaning as defined herein previously;
   or the corresponding cyclic hemiacetal
b1) reacting said polyhydroxy acid with an amine having the formula H₂NR², wherein R² has the same meaning as defined herein previously, under conditions that result in the coupling of the amine to the aldehyde moiety of the polyhydroxy acid to produce a reaction adduct and
b2) subsequently reducing the resulting reaction adduct to a secondary amine,
c) optionally isolating or purifying the secondary amine formed in step b2),
d) reacting the secondary amine of step b2) or c) with a suitable substrate under conditions that result in the formation of a compound of formula (I) wherein Y represents a group of formula (II) as defined herein previously.

In another aspect, the invention concerns a method of producing a compound of formula (I) as defined herein wherein Y represents a group of formula (III), the method comprising:
a) providing a polyhydroxy acid of formula (IV), 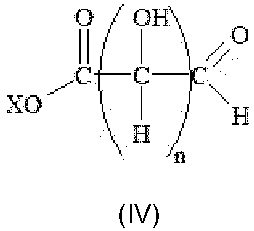 Wherein X and n have the same meaning as defined herein previously;
   or the corresponding cyclic hemiacetal
b1) reacting said polyhydroxy acid with an amine having the formula HNR²R³, wherein R² and R³ have the same meaning as defined herein previously and at least one of R² and R³ represents a surfactant tail group as defined herein previously, under conditions that result in the coupling of the amine to the aldehyde moiety of the polyhydroxy acid to produce a reaction adduct and
b2) subsequently reducing the resulting reaction adduct to a first compound of formula (I) wherein Y represents a group of formula (III) as defined herein previously,
c) optionally isolating or purifying the first compound formed in step b2),
d) optionally reacting the first compound of step b2) or c) with a suitable substrate under conditions that result in the formation of a second compound of formula (I) wherein Y represents a group of formula (III) as defined herein previously.

The reaction adduct resulting from step b1) will usually be an imine if a primary amine is employed in step b1) but without wishing to be bound by any considerations on the exact reaction mechanism, the reaction adduct may be any combination of the aldehyde and amine of step a) which will results in an amine after reduction, preferably by means of hydrogenation. In preferred embodiments the reaction adduct is an imine.

In a preferred embodiment of producing a compound of formula (I) as defined herein wherein Y represents a group of formula (II), step d) comprises reacting the secondary amine of step b2) or c) with a fatty acid ester having the structure R¹COOR⁴ wherein R¹ has the same meaning as defined herein previously and R⁴ represents a group that can suitably be displaced under the conditions applied in step d). In preferred embodiments of the invention R⁴ represents a group selected from methyl, ethyl, n-propyl, isopropyl, n-butyl, sec-butyl, isobutyl or *tert-*butyl, preferably from methyl, ethyl, n-propyl and isopropyl, more preferably from methyl and ethyl, most preferably it is methyl. In certain preferred embodiments R⁴ is a glycerol derivative such that R¹COOR⁴ is a fatty glyceride ester. The fatty glyceride ester may advantageously be sourced from a natural plant oil such as palm oil, palm kernel oil, soy oil, canola oil, tallow etc. Thus, in embodiments for producing a compound of formula (I) as defined herein wherein Y represents a group of formula (II), step d) comprises reacting the secondary amine formed in step b2) with a natural plant oil, preferably a natural plant oil selected from palm oil, palm kernel oil, soy oil, canola oil and tallow.

In an embodiment, a method is provided as defined herein, wherein the polyhydroxyacid is a uronic acid, preferably a uronic acid selected from the group consisting of hexuronic acids and penturonic acids; preferably from the group consisting of hexuronic acids; preferably from the group consisting of galacturonic acid, mannuronic acid, iduronic acid and guluronic acid; most preferably galacturonic acid. As already explained herein, it is particularly preferred in accordance with the invention that the polyhydroxy acid amides are produced from a renewable source, in particular from a biomass source. In accordance with one embodiment, suitable biomass sources include those containing substantial quantities of galacturonic acid, such as hemicellulosic and pectin rich biomass. Materials may accordingly be utilized that, at present, are still mainly considered by-products in various industries. In preferred embodiments of the invention, the hemicellulose and pectin rich biomass is sugar beet pulp, which constitutes the production side stream from the sugar beet industry. In alternative embodiments, the uronic acid is obtained from a plant source such as from citrus fruits, tomatoes, chicory, potatoes, pineapple, apple, cranberries, grapes, carrots and the like.

In accordance with a preferred embodiment of the invention, step b1) may comprise employing a suitable solvent. Examples of suitable solvents for carrying out step b1) include water and organic hydroxy solvents such as methanol, ethanol and glycerol. The use of water is particularly preferred. Step b1) may conveniently be carried out in a batch reactor, such as a continuous stirred tank reactor (CSTR). The reaction is typically carried out at a temperature and pressure and for a contact time sufficient to effect the formation of the target compounds. In certain embodiments of the invention, step b1) comprises subjecting the reaction mixture to temperatures within the range of 0 °C to 20 °C, preferably 0 °C to 15 °C, preferably 0 °C to 10 °C. Lower reaction temperatures are associated with less discoloration. In certain embodiments, step b1) comprises subjecting the reaction mixture to temperatures within the range of 20 °C to 100 °C, preferably 30 °C to 80 °C, preferably 40 °C to 70 °C. In certain embodiments of the invention, step b1) comprises subjecting the liquid reaction mixture to atmospheric pressure.

In accordance with a preferred embodiment of the invention, step b2) of the method described above may comprise a reduction process, preferably a hydrogenation process employing a suitable reagent and/or catalyst, such as sodium borohydride, sodium cyanoborohydride, sodium triacetoxyborohydride, platinum, ruthenium or a Raney nickel catalyst, preferably platinum, ruthenium or a Raney nickel catalyst, preferably a Raney nickel catalyst. In embodiments a hydrogenation catalyst, preferably a Raney nickel catalyst is employed in concentrations in the range of 0.1-100 wt.% by weight of the reaction adduct formed in step b1), preferably 0.1-80 wt.%, preferably 0.1-20 wt.%, most preferably 1-20 wt.%. In accordance with an embodiment of the invention, step b2) of the method described above comprises the addition of H₂ to the reaction mixture, e.g. by performing the reactor in a pressurized vessel containing a hydrogen atmosphere. In certain embodiments, the H₂ pressure employed is larger than larger than 2 bar, larger than 10 bar or larger than 100 bar. Examples of suitable solvents for carrying out step b2) include water; organic hydroxy solvents such as methanol, ethanol and glycerol; and combinations thereof. The use of water is particularly preferred. Step b2) may conveniently be carried out in a batch reactor, such as a continuous stirred tank reactor (CSTR). The reaction is typically carried out at a temperature and pressure and for a contact time sufficient to effect the formation of the target compounds. The reaction may be performed *"in situ",* i.e. employing the reaction mixture of step b1) without first isolating the adduct formed in step b1). In preferred embodiments, the adduct formed in step b1) is a stable imine or enamine which is isolated before the hydrogenation of step b2) is performed. In certain embodiments of the invention, the reaction is carried out under reflux conditions. In certain embodiments of the invention, step b2) comprises subjecting the reaction mixture to temperatures within the range of 70 °C to 200 °C, preferably 90 °C to 180 °C, preferably 110 °C to 160 °C. In certain embodiments step b2) comprises subjecting the reaction mixture to temperatures within the range of 30 °C to 80 °C, preferably 40 °C to 70 °C, preferably 50 °C to 60 °C. In certain embodiments of the invention, step b2) comprises subjecting the liquid reaction mixture to an atmosphere comprising hydrogen gas at a pressure within the range of 2-500 bar, preferably 30-300 bar, preferably 50-150 bar. In certain embodiments of the invention, step b2) comprises subjecting the liquid reaction mixture to an atmosphere comprising hydrogen gas at a pressure within the range of 1-50 bar, preferably 5-30 bar, preferably 10-20 bar. The reaction is carried out for a period of time sufficient to effect conversion under the chosen conditions. The reaction time can range from several minutes to a number of days, typically from 15 minutes to 50 hours, most preferably from 3-24 hours. In embodiments, carryover of unreacted amine starting material from step b1) into the reaction mixture of step b2) is limited such that the reaction mixture is substantially free of unreacted amine starting material from step b1). In preferred embodiments, the concentration of unreacted amine starting material from step b1) in the reaction mixture of step b2) is lower than 20 wt.% by weight of the reaction adduct formed in step b1), preferably lower than 10 wt.%, preferably lower than 5 wt.%, preferably lower than 1 wt.%. In accordance with certain embodiments of the invention, e.g. when a borohydride reagent is employed, the reaction may be performed at a temperature of lower than 25°C, lower than 15 °C, lower than 5 °C.

Conversion of the polyhydroxy acid into the target amine is preferably from about 20% to about 100% and most preferably from about 60% to about 100%. Selectivity for the target amine, is preferably from about 20% to 100% and most preferably from about 60% to 100%.

In accordance with preferred embodiments of the invention, step d) of the method described above may be carried out in the presence of a suitable catalyst. Suitable catalysts employed in step d) to produce a compound of formula (I) as defined herein wherein Y represents a group of formula (II) include trilithium phosphate, trisodium phosphate, tripotassium phosphate, tetrasodium pyrophosphate, tetrapotassium pyrophosphate, pentasodium tripolyphosphate, pentapotassium tripolyphosphate, lithium hydroxide, sodium hydroxide, sodium methoxide, potassium methoxide, potassium hydroxide, calcium hydroxide, lithium carbonate, sodium carbonate, potassium carbonate, disodium tartrate, dipotassium tartrate, sodium potassium tartrate, trisodium citrate, tripotassium citrate, sodium basic silicates, potassium basic silicates, sodium basic aluminosilicates, potassium basic aluminosilicates and mixtures thereof. The use of trisodium phosphate, tripotassium phosphate, sodium hydroxide, potassium hydroxide, calcium hydroxide, sodium carbonate, potassium carbonate, sodium methoxide, potassium methoxide and mixtures thereof is particularly preferred. The most highly preferred catalysts from this group are selected from sodium carbonate, potassium carbonate, sodium hydroxide, potassium hydroxide, and mixtures thereof. In embodiments of the invention, step d) of the method described above employs suitable quaternization reagents, e.g. alkyl halides such as iodomethane, to produce a compound of formula (I) as defined herein wherein Y represents a group of formula (III).

In embodiments, step d) of the method described above may be performed substantially or completely solvent free. In certain embodiments, suitable solvents include water, dimethylsulfoxide, dimethylformamide, acetone and organic hydroxy solvents such as methanol, ethanol and glycerol. In accordance with preferred embodiments of the invention, step d) of the method described above may be carried out in the presence of a phase transfer agent or emulsifier, preferably at a level of about 0.5 wt.% to about 95 wt.% by weight of the reaction mixture. In case a continuous synthesis is employed, higher amounts of phase transfer agent or emulsifier as preferred, such as more than 50 wt.% by weight of the reaction mixture, preferably more than 60 wt.%, preferably more than 70 wt.%. In case a batch synthesis is employed, lower amounts of phase transfer agent or emulsifier are preferred, such as 0.5-20 wt.% by weight of the reaction mixture, preferably 1-10 wt.%.

Step d) may conveniently be carried out in a batch reactor, such as a continuous stirred tank reactor (CSTR). The reaction is typically carried out at a temperature and pressure and for a contact time sufficient to effect the formation of the target compounds. In certain embodiments of the invention, the reaction is carried out under reflux conditions. In certain embodiments of the invention, (e.g. in order to facilitate melting of solid triglyceride reactants), step d) comprises subjecting the reaction mixture to temperatures within the range of 70 °C to 250 °C, preferably 120 °C to 200 °C, preferably 135 °C to 170 °C. Alternatively, in certain embodiments of the invention, step d) comprises subjecting the reaction mixture to temperatures within the range of 40 °C to 200 °C, preferably 55 °C to 170 °C, preferably 70 °C to 140 °C. In emodiments step d) is performed at room temperature. In certain embodiments of the invention, step d) comprises subjecting the liquid reaction mixture to a reduced pressure, within the range of 0.001-1 bar, preferably 0.01-0.9 bar. In embodiments step d) comprises subjecting the liquid reaction mixture to a reduced pressure of less than 0.1 bar, preferably less than 0.01 bar, preferably less than 0.001 bar. In certain embodiments the reaction is carried out at elevated pressure within the range of 2-500 bar, preferably 30-300 bar, preferably 50-150 bar. In certain embodiments the reaction is carried out at elevated pressure within the range of 1-50 bar, preferably 2-30 bar, preferably 2-10. Step d) may also be performed at atmospheric pressure. In accordance with some embodiments of the invention, step d) may be performed under an inert atmosphere, such as a nitrogen or argon atmosphere. The reaction is carried out for a period of time sufficient to effect conversion under the chosen conditions. The reaction time can range from several minutes to a number of days, typically from 15 minutes to 50 hours, most preferably from 3-24 hours. In certain embodiments step d) comprises subjecting the reaction mixture both to a period at reduced pressure and elevated temperature, and to a period at reduced pressure and less elevated temperature. In embodiments step d) comprises first subjecting the reaction mixture to a period at reduced pressure and elevated temperature, followed by subjecting the reaction mixture to a period at reduced pressure and less elevated temperature. In embodiments step d) comprises first subjecting the reaction mixture to a period of at least 30 seconds, preferably at least one minute, preferably at least 5 minutes, preferably at least 30 minutes at a pressure of less than 0.8 bar, preferably less than 0.5 bar, preferably less than 0.1 bar and a first temperature within the range of 80 °C to 200 °C, preferably 100 °C to 170 °C, preferably 120 °C to 140 °C, followed by subjecting the reaction mixture to a period of at least 30 seconds, preferably at least one minute, preferably at least 5 minutes, preferably at least 30 minutes at a pressure of less than 0.8 bar, preferably less than 0.5 bar, preferably less than 0.1 bar and a second temperature which is at least 20 °C lower than said first temperature, preferably at least 30 °C lower, preferably at least 40 °C lower. In certain embodiments step d) comprises subjecting the reaction mixture both to a period at elevated pressure and temperature, and to a period at reduced pressure and elevated temperature. In embodiments step d) comprises first subjecting the reaction mixture to a period at elevated pressure and temperature, followed by subjecting the reaction mixture to a period at reduced pressure and elevated temperature. This temperature and pressure scheme may facilitate the dissolution of reagents such as fatty acid derivatives, e.g. methyl esters of fatty acids or trigylcerides of fatty acids. In embodiments step d) comprises first subjecting the reaction mixture to a period of at least 30 seconds, preferably at least one minute, preferably at least 5 minutes, preferably at least 30 minutes at a pressure within the range of 1-50 bar, preferably 2-30 bar, preferably 2-10 bar pressure and a temperature within the range of 40 °C to 200 °C, preferably 55 °C to 170 °C, preferably 70 °C to 140 °C, followed by subjecting the reaction mixture to a period of at least 30 seconds, preferably at least one minute, preferably at least 5 minutes, preferably at least 30 minutes at a pressure of less than 0.8 bar, preferably less than 0.5 bar, preferably less than 0.1 bar and a temperature within the range of 40 °C to 200 °C, preferably 55 °C to 170 °C, preferably 70 °C to 140 °C. In embodiments, step d) comprises heating the reaction mixture by employing steam. The reaction is carried out for a period of time sufficient to effect conversion under the chosen conditions. The reaction time can range from several minutes to a number of days, typically from 30 minutes to 50 hours, most preferably from 3-24 hours. Conversion of the secondary amine into the target compound, i.e. the compound of formula (I), is preferably from about 20% to about 100% and most preferably from about 60% to about 100%. Selectivity for the target compound, is preferably from about 20% to 100% and most preferably from about 60% to 100%. In certain embodiments of the invention, step d) comprises the addition of a base to the reaction mixture, for example triethylamine or a basic anion exchange resin.

It is within the routine of those of average skill in the art to determine the appropriate conditions for carrying out the process and to optimize it in terms of yield, efficiency, etc.

In certain embodiments of the invention, steps b) and/or d) may be followed by a separation and/or isolation of the target intermediate or end product from the reaction mixture, by any suitable technique known by the person skilled in the art, such as chromatographic separation and/or crystallization.

A further aspect of the invention, concerns compounds and composition as obtained and/or obtainable by the methods defined herein. Such compounds and/or compositions may be the same or may differ in some aspect(s) from compounds and/or compositions as described herein elsewhere.

In a further aspect of the invention, the use of a compound of formula (I) as defined herein and/or a compound or composition obtainable by the methods defined herein as a surfactant or surface active component is provided.

Certain embodiments of the invention provide the use of a compound of formula (I) as defined herein and/or a compound or composition obtainable by the methods defined herein as a surfactant or surface active component in a product selected from the group consisting of laundry detergent products, dishwashing products, personal care products, hard surface cleaning products and agricultural products.

Certain embodiments of the invention provide the use of a compound of formula (I) as defined herein and/or a compound or composition obtainable by the methods defined herein for conferring and/or improving foaming properties, emulsifying properties and/or wetting properties in a product selected from the group consisting of laundry detergent products, dishwashing products, personal care products, hard surface cleaning products and agricultural products.

### Examples

### Example 1: Reductive amination of polyhydroxy acid aldehydes

The reductive amination of polyhydroxy acids aldehydes of formula (IV) or their corresponding cyclic hemiacetals was performed as follows.

A reaction mixture comprising alkylamine (2 equivalents), polyhydroxy acid aldehyde (1 equivalent) in ethanol is prepared at room temperature and stirred overnight. The solvent is removed *in vacuo* and the resulting solid adduct is mixed with methanol. 10 wt.% by weight of the adduct of a Ni catalyst supported on silica is added and the mixture is hydrogenated at about 17 bar and 50°C for 24 hours. The reaction mixture is filtered through a glass microfibre filter and optionally neutral silica gel or bleaching earth to remove nickel, the solvent is removed *in vacuo* and the *N*-alkyl-galacturonate is obtained.

### Example 2: Acylation procedure for sodium N-alkyl-galacturonates

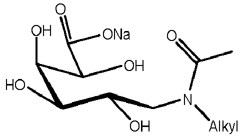

The acylation of sodium *N*-alkyl-galacturonates was performed as follows. Acetic anhydride (1.5 eq.) was added to the Sodium *N*-alkyl-galacturonate in ethanol and stirred overnight. The solution was neutralized with Dowex OH⁻ and the ethanol was removed under reduced pressure.

Compounds were purified by crystallization by adding a small amount of acetone and hexane.

### Example 3: Acylation procedure for sodium N-alkyl-galacturonates

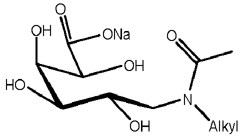

An alternative procedure for the acylation of sodium *N*-alkyl-galacturonates was as follows. Triethylamine (1 eq.) is added to a solution of Sodium *N*-alkyl-galacturonate in DMF under a N₂ atmosphere. A concentrated solution of acetic anhydride (1 eq.) is added and the mixture is stirred at room temperature for 8 hours. The solvent is then removed under reduced pressure.

### Example 4: Quaternisation procedure for sodium N-alkyl-galacturonates

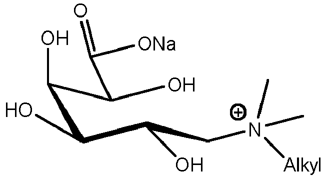

The quaternisation of sodium *N*-alkyl-galacturonates was performed as follows. A solution of Sodium *N*-alkyl-galacturonate (1eq., 2mmol) in DMSO (25 mL) under N₂ is treated with iodomethane (6 eq., 12 mmol). The mixture is stirred at room temperature for 5 hours. The solvent is then removed under reduced pressure.

### Example 5: Synthesis of Sodium N-octylamine galacturonate

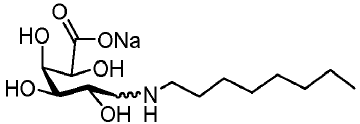

A mixture of galacturonic acid (780 mg, 4.02 mmol) and octylamine (571 mg, 84.42 mmol) in methanol was stirred for 24h at rt. Then, the mixture was concentrated *in vacuo,* dissolved in methanol and the pH was checked and brought to eight. The reaction mixture was cooled to 0 °C, and NaBH₄ (304 mg, 8.04 mmol) was added stepwise. The solution was left stirring for 2h, and then the pH was lowered to 2-3 with 1 M hydrochloric acid. Subsequently the mixture was stirred with molecular sieves 4Å, and NaOH pellets (239 mg, 4.42 mmol) for 24h at rt and afterwards refluxed for 1 h. The reaction mixture was filtrated, and concentrated *in vacuo* to obtain Sodium N-octylamine galacturonate (430 mg, 1.3 mmol, 32%).

### Example 6: synthesis of Sodium N-methylamine galacturonate

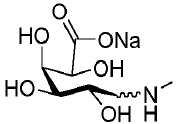

A mixture of galacturonic acid (20 g, 0.10 mol) and methylamine (2.7 g, 0.21 mol) in methanol was stirred for 24 h at rt. Then, the mixture was concentrated *in vacuo,* dissolved in methanol and the pH was checked and brought to eight. The reaction mixture was cooled to 0 °C, and NaBH₄ (4.3 g, 0.11 mmol) was added stepwise. The solution was left stirring for 2h, and then the pH was lowered till 2-3 with 1 M hydrochloric acid. Subsequently the mixture was stirred with molecular sieves 4Å, and NaOH (4.5 g 0.11 mmol) for 24 h at rt and afterwards refluxed for 1 h. The reaction mixture was filtrated, and concentrated *in vacuo* to obtain Sodium *N*-methylamine galacturonate (16.03 g, 69.1 mmol, 69.1%).

### Example 7: synthesis of Sodium N-methyl-lauryl-amide galacturonate

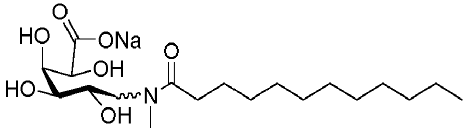

A slurry of Sodium N-Methylamine galacturonate (2 g, 8.6 mmol), methyl laurate (2.59 mL 2.254 g, 10.52 mmol) and methanol (0.84 g, 20 wt% based on reactants) was heated to 80°C under a N₂ atmosphere. When the reaction mixture reached a temperature of 80°C, sodium methoxide (52 mg of 25 % in methanol) was added. The reaction mixture was stirred for 2 h at 80°C, and was continued for an additional 3 h under reflux. The reaction mixture was cooled to rt, and then concentrated *in vacuo.*

## Claims

1. Compound of formula (I): 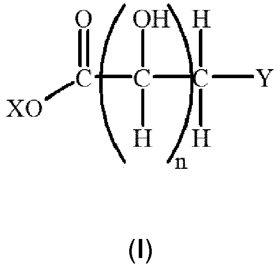 wherein X represents hydrogen or a cation; n is 2, 3 or 4 and Y represents a group of formula (II) or formula (III): 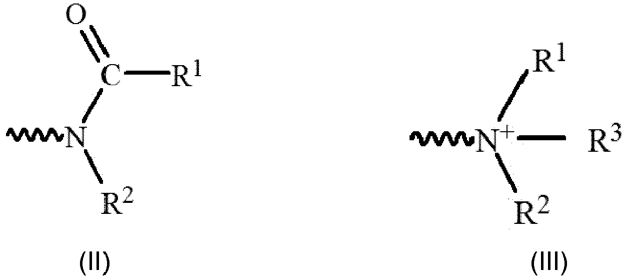 wherein R¹, R² and R³ independently represent hydrogen, a C₁-C₄ alkyl, a C₁-C₄ alcohol or a surfactant tail group, with the proviso that at least one of R¹, R² and R³ represents a surfactant tail group.

2. Compound according to claim 1, wherein n is 3 or 4, preferably n is 4.

3. Compound according to any one of the preceding claims, wherein the suitable surfactant tail group represented by at least one of R¹, R² and R³ is the residue of a C₆-C₂₄ fatty acid, preferably the residue of a fatty acid selected from the group consisting of caproic acid, caprylic acid, capric acid, lauric acid, lauroleic acid, myristic acid, myristoleic acid, palmitic acid, palmitoleic acid, stearic acid, oleic acid, linoleic acid, linolenic acid, ricinoleic acid, arachidic acid, arachidonic acid, gadoleic acid, erucic acid, behenic acid and lignoceric acid.

4. Compound according to any one of the preceding claims, wherein the moiety represented by formula (I'): 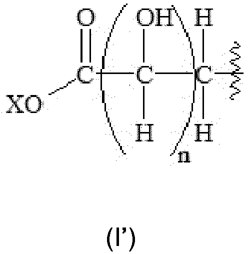 represents a uronic acid residue, preferably the residue of a uronic acid selected from the group consisting of hexuronic acids and penturonic acids; preferably from the group consisting of hexuronic acids; preferably from the group consisting of galacturonic acid, iduronic acid, mannuronic acid and guluronic acid; most preferably galacturonic acid.

5. Compound according to any one of the preceding claims, wherein R² represents a C₁-C₃ alkyl.

6. Product selected from the group consisting of laundry detergent products, dishwashing products, personal care products, hard surface cleaning products, comprising a compound as defined in any one of the preceding claims.

7. A method of producing a compound, the method comprising:
a) providing a polyhydroxy acid of formula (IV), 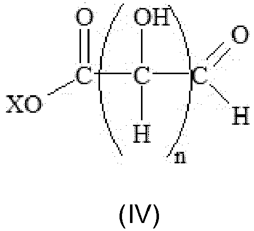 wherein X and n have the same meaning as defined in any one of claims 1-6; or the corresponding cyclic hemiacetal
b1) reacting said polyhydroxy acid with an amine having the formula H₂N-R², wherein R² has the meaning as defined in any one of claims 1-6, under conditions that result in the coupling of the amine to the aldehyde moiety of the polyhydroxy acid to produce a reaction adduct and
b2) subsequently reducing the resulting reaction adduct to a secondary amine,
c) optionally isolating or purifying the secondary amine formed in step b2),
d) reacting the secondary amine of step b2) or c) with a suitable substrate under conditions that result in the formation of a compound of formula (I) wherein Y represents a group of formula (II) as defined in any one of claims 1-6.

8. A method of producing a compound, the method comprising:
a) providing a polyhydroxy acid of formula (IV), 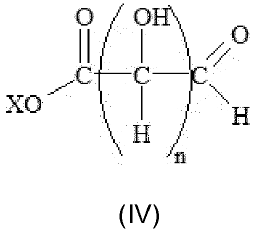 Wherein X and n have the same meaning as defined herein previously;
or the corresponding cyclic hemiacetal
b1) reacting said polyhydroxy acid with an amine having the formula HNR²R³, wherein R² and R³ have the same meaning as defined any one of claims 1-6 and at least one of R² and R³ represents a surfactant tail group as defined any one of claims 1-6, under conditions that result in the coupling of the amine to the aldehyde moiety of the polyhydroxy acid to produce a reaction adduct and
b2) subsequently reducing the resulting reaction adduct to a first compound of formula (I) wherein Y represents a group of formula (III) as defined in any one of claims 1-6,
c) optionally isolating or purifying the first compound formed in step b2),
d) optionally reacting the first compound of step b2) or c) with a suitable substrate under conditions that result in the formation of a second compound of formula (I) wherein Y represents a group of formula (III) as defined in any one of claims 1-6.

9. Method according to any one of claims 7 or 8 wherein the polyhydroxy acid is a uronic acid, preferably a uronic acid selected from the group consisting of hexuronic acids and penturonic acids; preferably from the group consisting of hexuronic acids; preferably from the group consisting of galacturonic acid, mannuronic acid, iduronic acid and guluronic acid; most preferably galacturonic acid.

10. Method according to any one of claims 7 or 9 wherein step d) comprises reacting the secondary amine of step b2) or c) with a fatty acid ester having the structure R¹COOR⁴ wherein R¹ has the meaning as defined in claim 1 and R⁴ represents a group that can suitably be displaced under the conditions applied in step d).

11. Method according to claim 10 wherein the fatty acid ester is an ester of a fatty acid selected from the group consisting of a C₆-C₂₄ fatty acid, preferably the residue of a fatty acid selected from the group consisting of caproic acid, caprylic acid, capric acid, lauric acid, lauroleic acid, myristic acid, myristoleic acid, palmitic acid, palmitoleic acid, stearic acid, oleic acid, linoleic acid, linolenic acid, ricinoleic acid, arachidic acid, arachidonic acid, gadoleic acid, erucic acid, behenic acid and lignoceric acid.

12. Method according to any one of claims 7, 9, 10 and 11 wherein R² is a methyl group.

13. Compound obtainable by the method as defined in any one of claims 7-12.

14. Use of a compound as defined in any one of claims 1-6 and 13, as a surfactant.

15. Use of a compound as defined in any one of claims 1-6 and 14, as an anionic surfactant in a product selected from the group consisting of pharmaceuticals, neutraceuticals, feed, food, cosmetics, detergents, fabric softeners, soaps, paints, adhesives, inks, anti-fogs, agrochemical products, herbicides, insecticides, biocides, cosmetics, shampoos, hair conditioners, toothpastes, coatings, drilling fluids, oilfield chemicals, emulsion polymerization systems and ferrofluids.

16. Use of a compound as defined in any one of claims 1-6 and 14, for conferring and/or improving foaming properties, emulsifying properties and/or wetting properties in a product selected from the group consisting of laundry detergent products, dishwashing products, personal care products, hard surface cleaning products and agricultural products.
